# EUROPEAN PATENT APPLICATION

(11) **EP 2 163 252 A1**
(43) Date of publication of application: **17.03.2010**
(21) Application number: 08752895.6
(22) Date of filing: 16.05.2008
(51) Int. Cl.: A61K 36/48, A23K 1/14, A23K 1/16, A23L 1/30, A23L 1/302, A23L 1/305, A61K 31/07, A61K 31/122, A61K 31/198, A61K 31/221, A61K 31/35, A61K 31/353, A61K 31/355, A61K 31/36, A61K 31/375, A61K 45/00, A61P 3/00, A61P 3/02, A61P 3/04, A61P 3/06, A61P 3/10

(54) **COMPOSITION CONTAINING LICORICE-DERIVED POLYPHENOL**

(30) Priority: 17.05.2007 JP 2007131779; 18.07.2007 US 950432 P
(71) Applicant: Kaneka Corporation, Kita-ku Osaka-shi, Osaka 530-8288 (JP)
(72) Inventor: SAKAMOTO, Hirokazu, Takasago-shi Hyogo 676-8688 (JP); TOMINAGA, Yuji, Takasago-shi Hyogo 676-8688 (JP); KITAMURA, Shiro, Takasago-shi Hyogo 676-8688 (JP); YOKOTA, Shinichi, Takasago-shi Hyogo 676-8688 (JP)
(74) Representative: Siegert, Georg
(86) International application number: PCT/JP2008/059062
(87) International publication number: WO 2008/143182

(57) **Abstract**

A composition containing a licorice polyphenol and other active ingredient (antioxidant ingredient, polyphenol other than licorice polyphenol or a component relating to lipid absorption or metabolism) is highly safe, can be produced easily, and can be utilized for foods and drinks such as health food, food with health claims (specified health food, food with nutrient function claims) and the like, pharmaceutical products, quasi-drugs, cosmetics and the like. In addition, since the composition has a body fat accumulation suppressing effect, a body fat decomposition promoting effect or energy production promoting effect, it is useful for the treatment of obesity, metabolic syndrome and the like.

## Description

### Technical Field

The present invention relates to a composition comprising licorice polyphenol, which can be used for foods and drinks such as health food, food with health claims (specified health food, food with nutrient function claims) and the like, pharmaceutical products, quasi-drugs, cosmetics, animal drugs, pet foods, feeds or prey feeds, and the like.

### Background Art

Licorice is a plant belonging to the genus Fabaceae Glycyrrhiza widely distributed in the People's Republic of China, Europe, Russian Federation, Republic of Afghanistan, Iran, Islamic Republic of Pakistan and the like. The roots thereof and the like have a long history of ingestion where they were utilized as a food or a crude drug. Since glycyrrhizin (glycyrrhizic acid ), which is the main component of a water extract of licorice, has superior pharmacological actions such as an anti-inflammatory action, an antitumor action, an antiallergic action and the like, it has been widely utilized for foods, pharmaceutical products, cosmetics and the like. In addition, since glycyrrhizin is about 200 times as sweet as sucrose, it is also used as a sweetener. Licorice is used for food additives and foods in Japan. In the US, licorice was registered as a GRAS (Generally Recognized As Safe) food by FDA in 1985, approved as a cancer preventive food in the Designer Foods 1990, and ingestion thereof was recommended. In Europe, licorice is taken daily as licorice sweets such as licorice candy and the like.

On the other hand, a licorice hydrophobic component extracted from licorice or a licorice water extract residue with an organic solvent and the like has been reported to show many useful actions such as an antioxidant action, an antibacterial action, an enzyme inhibitory action, an antitumor action, an antiallergic action, an antiviral action and the like. The main varieties of licorice include Glycyrrhiza glabra, Glycyrrhiza uralensis (G. uralensis), Glycyrrhiza inflata (G. inflate) and the like. Each variety includes glycyrrhizin (glycyrrhizic acid), which is a hydrophilic component. In addition, licorice contains a large amount of prenylflavonoid as licorice polyphenol, and particularly contains a variety-specific flavonoid as a hydrophobic component (non-patent reference 1). The variety-specific flavonoid may also be used for identification of the licorice variety.

Recently, it has been found that, of the extracts obtained from licorice, a licorice hydrophobic extract containing licorice polyphenol and having an ultratrace glycyrrhizin content has a visceral fat reducing action and is useful for the prophylaxis and/or improvement of metabolic syndrome (multiple risk factor syndrome)(patent reference 1). In addition, it has been reported that a licorice hydrophobic extract has a lipase inhibitory action (patent reference 2), has a cholesterol absorption inhibitory action (patent reference 2), a component thereof has a PPARγ ligand activity (patent references 3, 4 and 5) and the like.

Licorice is used as a crude drug containing glycyrrhizin alone as an active ingredient, or in combination with a plurality of crude drugs. However, a report positively referring to a composition comprising a combination of a licorice hydrophobic extract substantially containing a small amount of glycyrrhizin and other polyphenol, flavonoid component and the like has not been found.
patent reference 1: WO 02/47699
patent reference 2: WO2005/110400
patent reference 3: WO03/037316
patent reference 4: WO2006/085562
patent reference 5: WO2007/060992
non-patent reference 1: Progress in the Chemistry of Organic Natural Products, Vol.73, (1998)

### Disclosure of the Invention

### Problems to be Solved by the Invention

The present inventors have studied with the aim to provide a composition comprising licorice polyphenol, which is highly safety, can be produced easily, and can be utilized for foods and drinks such as health food, food with health claims (specified health food, food with nutrient function claims) and the like, pharmaceutical products, quasi-drugs, cosmetics and the like.

### Means of Solving the Problems

The present inventors have found that a more preferable composition can be provided by appropriately combining licorice polyphenol with an antioxidant ingredient, polyphenol other than licorice polyphenol or a component relating to lipid absorption or metabolism (e.g., a lipid absorption inhibitory component, a component modifying lipid metabolism in cell and the like). In addition, they have found that the composition of the present invention is effective for the prophylaxis or treatment of body fat accumulation, obesity, metabolic syndrome and the like, since it provides effects of suppressing body fat accumulation, promoting decomposition of body fat and promoting energy production. Accordingly, the present invention provides the following.

[1] A composition comprising licorice polyphenol and an antioxidant ingredient.
[2] The composition of [1], wherein the antioxidant ingredient is at least one kind selected from the group consisting of α-lipoic acid, glutathione, astaxanthin, lycopene, taurine, methionine, S-adenosylmethionine, cysteine, cystine, thiol compound, reducing sugar, sesamin, capsaicin, citric acid, pycnogenol, fucoxanthin, lignan, piperine, policosanol, anthocyanin, proanthocyanidin, chlorogenic acid, vitamin C, vitamin A, vitamin E and carotenoid.
[3] A composition comprising licorice polyphenol and polyphenol other than licorice polyphenol.
[4] The composition of [3], wherein the polyphenol other than licorice polyphenol is at least one kind selected from the group consisting of genistein, daidzein, quercetin, rutin, catechin, epigallocatechin gallate, hesperidin, nobiletin, tyrosol, hydroxytyrosol, oleuropein, naringenin, caffeic acid, apple polyphenol, tea polyphenol and gallic acid.
[5] A composition comprising licorice polyphenol and a component relating to absorption or metabolism of lipid.
[6] The composition of [5], wherein the component relating to the absorption or metabolism of lipid is a lipid absorption inhibitory component.
[7] The composition of [6], wherein the lipid absorption inhibitory component is at least one kind selected from the group consisting of lactobacillus, yeast, polysaccharides, dietary fiber, oligosaccharide, inulin, chitin, chitosan, phytosterol, pectin, glucosamine, N-acetylglucosamine, hyaluronan, chondroitin sulfate, sodium alginate and tannin.
[8] The composition of [5], wherein the component relating to the absorption or metabolism of lipid is a component modifying lipid metabolism in cell.
[9] The composition of [8], wherein the component modifying lipid metabolism in cell is at least one kind selected from the group consisting of carnitine, capsaicin, malic acid, hydroxycitric acid, acetic acid, propionic acid, citric acid, sesamin, pyruvic acid, curcuminoid, gingerol, pycnogenol, resveratrol, soybean protein, conjugated linoleic acid, catechin, caffeine, fucoxanthin, diacylglycerol, glucomannan, lignan, fructooligosaccharide, piperine, policosanol, chrome, zinc, vanadium, phospholipid and highly-unsaturated fatty acid.
[10] The composition of [8], wherein the component modifying the lipid metabolism in cell is at least one kind selected from the group consisting of chili pepper, tea, fermented tea, coffee, pepper, garcinia cambogia, ginger, blueberry, citrus, grape and extracts thereof.
[11] The composition of any one of [1] - [10], further comprising vitamin or a vitamin-like component.
[12] The composition of [11], wherein the vitamin or vitamin-like component is at least one kind selected from the group consisting of vitamin B, vitamin K, folic acid and inositol.
[13] The composition of any one of [1] - [12], further comprising an amino acid or an amino acid derivative.
[14] The composition of [13], wherein the amino acid or amino acid derivative is at least one kind selected from the group consisting of alanine, valine, leucine, isoleucine, glycine, proline, glutamine, glutamic acid, asparagine, aspartic acid, arginine, lysine, citrulline, ornithine and derivatives thereof.
[15] The composition of any one of [1] - [14], comprising licorice polyphenol in the form of a licorice hydrophobic extract.
[16] The composition of any one of [1] - [15], wherein the licorice polyphenol comprises at least glabridin.
[17] The composition of [16], wherein the content of glycyrrhizin in the composition is not more than the content of glabridin.
[18] The composition of any one of [1] - [17], further comprising medium chain fatty acid triglyceride.
[19] The composition of any one of [1] - [18], which is a body fat accumulation suppressor.
[20] The composition of any one of [1] - [18], which is a body fat decomposition promoter.
[21] The composition of any one of [1] - [18], which is an energy production promoter.
[22] The composition of any one of [1] - [21], which is used for the treatment of obesity, metabolic syndrome, diabetes, hyperlipidemia or hypertension.
[23] The composition of any one of [1] - [22], which is used for a food, drink, functional food, nutritional supplement, pharmaceutical product, quasi-drug, cosmetic, animal drug, pet food, feed or prey feed.
[24] The composition of any one of [1] - [23], which is used in combination with a pharmaceutical product to be used for the treatment of obesity, metabolic syndrome, diabetes, hyperlipidemia or hypertension.
[25] The composition of any one of [1] - [24], which is a preparation for oral administration.
[26] A method for promoting an energy production, comprising administering an effective amount of the composition of any one of [1] - [25] to a subject.
[27] A method for the prophylaxis, improvement or treatment of obesity, metabolic syndrome, diabetes, hyperlipidemia or hypertension, comprising administering an effective amount of the composition of any one of [1] - [25] to a subject.
[28] A method for becoming slim, comprising administering an effective amount of the composition of any one of [1] - [25] to a subject.
[29] Use of licorice polyphenol and an antioxidant ingredient for the production of the composition of any one of [1] - [25] for suppressing body fat accumulation, promoting body fat decomposition or promoting energy production.
[30] Use of licorice polyphenol and polyphenol other than licorice polyphenol for the production of the composition of any one of [1] - [25] for suppressing body fat accumulation, promoting body fat decomposition or promoting energy production.
[31] Use of licorice polyphenol and a component relating to absorption or metabolism of lipid for the production of the composition of any one of [1] - [25] for suppressing body fat accumulation, promoting body fat decomposition or promoting energy production.

### Effect of the Invention

The composition of the present invention comprising a combination of licorice polyphenol and other active ingredient(s) can be used as an agent for the prophylaxis or improvement of obesity and metabolic syndrome etc. caused by the obesity. In addition, since the composition is a combination of a plant-derived component eaten before and a useful component taken every day, it causes a few side effects and can be ingested for a long time.

### Best Mode for Carrying Out the Invention

The mode of embodiment of the present invention is explained in detail in the following.

The composition of the present invention comprises licorice polyphenol combined with an antioxidant ingredient, polyphenol other than licorice polyphenol or a component relating to absorption or metabolism of lipid (e.g., a lipid absorption inhibitory component, a component modifying lipid metabolism in cell and the like) (hereinafter to be sometimes referred generally to the composition of the present invention).

The licorice polyphenol in the present invention means a polyphenol component in licorice. In the present invention, licorice from which licorice polyphenol is derived is not particularly limited as long as it belongs to the genus Glycyrrhiza. Specific examples thereof include Glycyrrhiza uralensis (G. uralensis), Glycyrrhiza inflata (G. inflate), Glycyrrhiza glabra (G. glabra), Glycyrrhiza eurycarpa (G. eurycarpa), Glycyrrhiza aspera (G. aspera) and the like. Preferred are G. uralensis, G. inflata, G. glabra and the like, and more preferred is G. glabra.

The licorice polyphenol to be contained in the composition of the present invention is not particularly limited as long as it is a polyphenol component contained in the above-mentioned licorice. Specific examples thereof include glycycoumarin, glycyrol, glycyrin, liquiritigenin, glicoricone, glabridin, glabrene, glabrol, 3'-hydroxy-4'-O-methylglabridin, 4'-O-methylglabridin, glyurallin B, licocoumarone, gancaonin I, dehydroglyasperin D, echinatin, isolicoflavonol, dehydroglyasperin C, glyasperin B, glycyrrhisoflavanone, lupiwighteone, glyasperin D, semilicoisoflavone B and the like. The composition of the present invention contains at least one kind of licorice polyphenol from among such licorice polyphenol components. Among these, the composition of the present invention preferably contains at least any one kind of glabridin, glabrene, glabrol, 3'-hydroxy-4'-O-methylglabridin, 4'-O-methylglabridin and the like, and more preferably contains at least glabridin.

Polyphenol is a compound generally having a structure containing a plurality of phenolic hydroxyl groups, and means any compound or a derivative thereof, wherein plural hydroxyl groups are bonded to the benzene ring. The amount of polyphenol can be measured by, for example, colorimetric methods such as iron tartrate method, Persian blue method, Folin-Ciocalteu assay, Folin-Denis assay and the like, measurement per components by HPLC and the like, and any measurement method can be used. For the measurement of the whole amount of polyphenol, Folin-Denis assay or Folin-Ciocalteu assay is often used. In the case of Folin-Denis assay or Folin-Ciocalteu assay, a standard substance is used to draw an analytical curve of the standard substance, and the whole amount of polyphenol is determined by conversion based on the standard substance. In case of licorice hydrophobic extract, G. glabra, for example, the glabridin can be used as the standard substance. To be specific, for example, the content of the licorice polyphenol component in the composition of the present invention can be determined on the basis of glabridin by the method described in the below-mentioned Examples.

Most of licorice polyphenol has an anti-oxidative action and many are dissolved in medium chain fatty acid. The anti-oxidative ability of the composition of the present invention can be measured by, for example, an active oxygen and/or free radical eliminating action. In the composition of the present invention, when the anti-oxidative ability in the co-presence of licorice polyphenol and other active ingredient exceeds that of the two alone, the composition of the present invention can synergistically enhance the anti-oxidative ability.

The licorice polyphenol to be contained in the composition of the present invention may be licorice polyphenol itself as a pure product (for example, isolated and purified product, a reagent, a chemically synthesized product and the like). When isolated and purified from a naturally occurring substance, any known polyphenol component in licorice may be used even when it is not derived from licorice. In addition, a composition wherein licorice polyphenol is in the form of a licorice hydrophobic extract is also the composition of the present invention. From the aspects of production cost and the like, the composition of the present invention is preferably produced using a licorice hydrophobic extract containing licorice polyphenol as a main component. In this case, the licorice hydrophobic extract to be used can be obtained from licorice of, for example, G. glabra, G. uralensis, G. inflata and the like.

The licorice hydrophobic extract in the present invention is an extract solution obtained by extracting a hydrophobic component of licorice, or an extract obtained by removing the extraction solvent therefrom. When the licorice hydrophobic extract in the present invention is obtained from licorice, the method thereof is not particularly limited. For example, it can be obtained by removing a hydrophobic component from licorice or a powder thereof, or a licorice cell culture and the like by extraction using an organic solvent, and the like. Alternatively, a hydrophilic component in licorice is removed by extraction using water or aqueous alkali solution, and a hydrophobic component in the licorice residue with or without drying is removed by extraction using an organic solvent. Alternatively, the hydrophobic extract once extracted by the above-mentioned method is again extracted with a different kind of organic solvent.
The organic solvent to be used here is preferably one permitted to be used for the production and processing of pharmaceutical products, foods, food additives and the like. Examples of the organic solvent include alcohols (e.g., ethanol), esters (e.g., ethyl acetate), ketones (e.g., acetone), hydrocarbons (e.g., hexane) and the like, fats and oils (e.g., medium chain fatty acid triglyceride) and the like. Preferred are alcohols, ketones and fats and oils, and specifically, ethanol, acetone, medium chain fatty acid triglyceride and the like are preferably used. These solvents may be used alone or in a mixture of two ore more kinds thereof. In addition, water-containing solvents thereof may also be used. To suppress the content of the below-mentioned glycyrrhizin, the water-containing ratio of an extraction solvent is preferably set low.
The licorice hydrophobic extract in the present invention may be directly used as an extract obtained by extraction using the aforementioned organic solvent. It may be further purified crudely or purified by purification steps such as column treatment, deodorization treatment, decolorization treatment and the like. When the composition of the present invention contains a licorice hydrophobic extract as licorice polyphenol, the licorice hydrophobic extract contains licorice polyphenol as a main component at, for example, a proportion of preferably not less than 60 wt%, more preferably not less than 70 wt%, of the aforementioned licorice polyphenol.

In the composition of the present invention, the content of glycyrrhizin in the composition is preferably not more than the content of the licorice polyphenol component in the composition, more preferably not more than the content of glabridin in the licorice polyphenol component. In the licorice hydrophobic extract, components other than licorice polyphenol, for example, glycyrrhizin, which is a hydrophilic component, may be contained depending on the extraction conditions. When a licorice hydrophobic extract is used for the composition of the present invention, the content of glycyrrhizin is preferably smaller from the aspect of safety on long-term ingestion. Therefore, it is preferable to use a licorice hydrophobic extract substantially free of glycyrrhizin or having a low content thereof.

While the content of licorice polyphenol in the composition of the present invention is not particularly limited, it is preferably not less than 0.1 wt%, and more preferably not less than 1 wt%.
While the content of the licorice hydrophobic extract in the composition of the present invention is not particularly limited, it is preferably not less than 0.1 wt%, more preferably not less than 1 wt%, particularly preferably not less than 3 wt%. The upper limit of the content of the licorice polyphenol or licorice hydrophobic extract in the composition is not particularly limited, and the licorice polyphenol component is preferably contained in a large amount. To contain other active ingredients in necessary amounts, it is preferably not more than 99 wt%, more preferably not more than 90 wt%.

In one embodiment of the composition of the present invention, the composition contains licorice polyphenol and an antioxidant ingredient. The antioxidant ingredient in the present invention is a component that suppresses oxidation of protein, lipid, polyphenol and flavonoid, or a component that eliminates active oxygen and/or free radical. The antioxidant ingredient is a useful component for suppressing product degradation and odor and protecting living organisms from disorders caused by active oxygen or free radical. Examples of the antioxidant ingredient in the present invention include, but are not particularly limited to, α-lipoic acid, glutathione, astaxanthin, lycopene, taurine, methionine, S-adenosylmethionine, cysteine, cystine, thiol compounds such as coenzyme A and the like, reducing sugar, sesamin, capsaicin, citric acid, pycnogenol, fucoxanthin, lignan, piperine, policosanol, anthocyanin, proanthocyanidin, chlorogenic acid, vitamin C, vitamin A, vitamin E and carotenoid and the like. Among these, α-lipoic acid, glutathione, astaxanthin, lycopene, taurine, methionine, S-adenosylmethionine, thiol compounds such as coenzyme A and the like, sesamin, capsaicin, citric acid, pycnogenol, fucoxanthin, piperine, policosanol, anthocyanin, proanthocyanidin, chlorogenic acid, vitamin C, vitamin A, vitamin E and carotenoid are preferable, and α-lipoic acid, glutathione, astaxanthin and S-adenosylmethionine are particularly preferable.
The above-mentioned antioxidant ingredients may be used alone or in a mixture of two or more kinds thereof. The reducing sugar here means one having a reducing power, and includes all monosaccharides and malt sugar and lactose in disaccharides. Sesamin, capsaicin, citric acid, pycnogenol, fucoxanthin, lignan, piperine and policosanol also have the below-mentioned action of modifying lipid metabolism in cell.

In another embodiment of the composition of the present invention, the composition contains licorice polyphenol and polyphenol other than licorice polyphenol. Examples of the polyphenol other than licorice polyphenol include genistein, daidzein, quercetin, rutin, catechin, epigallocatechin gallate, hesperidin, nobiletin, tyrosol, hydroxytyrosol, oleuropein, naringenin, caffeic acid, apple polyphenol, tea polyphenol, gallic acid and the like. Preferred are genistein, daidzein, quercetin, rutin, catechin, epigallocatechin gallate, hesperidin, nobiletin, naringenin, caffeic acid, apple polyphenol and tea polyphenol. These polyphenols may be used alone or in a mixture of two or more kinds thereof. Genistein here is polyphenol contained in leguminous plants such as soybean and the like.

In still another embodiment of the composition of the present invention, the composition contains licorice polyphenol and a component relating to absorption or metabolism of lipid. Examples of the component relating to absorption or metabolism of lipid include a lipid absorption inhibitory component and a component modifying lipid metabolism in cell.

The lipid absorption inhibitory component in the present invention is a component that inhibits absorption of lipid from the intestine. Specific examples include lactobacillus, yeast, polysaccharides, dietary fiber, oligosaccharide, inulin, chitin, chitosan, pectin, sodium alginate, glucosamine, N-acetylglucosamine, hyaluronan, chondroitin sulfate, tannin, phytosterol and the like. Preferred are polysaccharides, dietary fiber, oligosaccharide, tannin and phytosterol. These components may be used alone or in a mixture of two or more kinds thereof.

The component modifying lipid metabolism in cell in the present invention is a component that promotes or delays metabolism of lipids and carbohydrates in living organisms and the like. Specific examples thereof include carnitine, capsaicin, malic acid, hydroxycitric acid, acetic acid, propionic acid, citric acid, sesamin, pyruvic acid, curcuminoid, gingerol, pycnogenol, resveratrol, soybean protein, conjugated linoleic acid, catechin, caffeine, fucoxanthin, diacylglycerol, glucomannan, lignan, fructooligosaccharide, piperine, policosanol, chrome, zinc, vanadium, phospholipid, highly-unsaturated fatty acids such as DHA, EPA etc., and the like, chili pepper, tea, fermented tea, coffee, pepper, garcinia cambogia, ginger, blueberry, citrus, grape, extracts thereof, and the like. Preferred are carnitine, capsaicin, acetic acid, sesamin, curcuminoid, gingerol, pycnogenol, resveratrol, soybean protein, conjugated linoleic acid, catechin, caffeine, fucoxanthin, diacylglycerol, lignan, piperine, policosanol, chrome, zinc, vanadium, phospholipid, highly-unsaturated fatty acids such as DHA, EPA etc., and the like, chili pepper, tea, fermented tea, coffee, pepper, garcinia cambogia, ginger, blueberry, citrus, grape and extracts thereof, and more preferred are carnitine, sesamin, pycnogenol, conjugated linoleic acid, catechin caffeine and vanadium. These components may be used alone or in a mixture of two or more kinds thereof.
The sugar metabolism system including glycolytic system and gluconeogenesis system responsible for glucose decomposition and synthesis is deeply involved in the fatty acid metabolism system. For example, since NADPH₂ necessary for fatty acid synthesis is supplied from the glucose glycolytic system, inhibition of glucose glycolytic system, or inhibition of glucose-6-phosphoric acid dehydrogenase or malic enzyme also inhibits fatty acid synthesis. Accordingly, a component influential on the sugar metabolism system is also included in the component modifying the lipid metabolism in the present invention.

While the content of the aforementioned other active ingredients in the composition of the present invention is not particularly limited, in expectation of a synergistic effect with licorice polyphenol, the weight ratio of licorice polyphenol:other active ingredients is preferably within the range of 1:100 - 10000:1, more preferably 1:10 - 1000:1, and particularly preferably 1:5 - 10:1.

The composition of the present invention may further contain vitamin or vitamin-like component. The vitamin or vitamin-like component refer to a compound in a trace amount which plays an important role for the metabolism in the body but cannot be produced in the body, and a compound outside such definition but plays an important role in the body. Examples of the vitamin in the present invention include water-soluble vitamins such as vitamin B (group) (e.g., vitamins B1, B2, B6, B12, folic acid, niacin, biotin, pantothenic acid and the like), vitamin C (L-ascorbic acid) and the like; fat-soluble vitamins (e.g., vitamins A, D, E, K and the like); and the like. Vitamins having a biological regulatory function with a trace amount thereof are considered to perform biological regulation in combination with polyphenol and a component called flavonoid, which have a biological regulatory function, and a combination of vitamin and licorice polyphenol is effective.
In addition, examples of the vitamin-like component include, for example, choline, inositol, p-aminobenzoic acid, pangamic acid, vitamin U, nicotinic acid amide, ester derivatives of vitamins C and E and the like, and further, coenzymes such as FAD, NADH, NADPH and the like, phosphocreatine, ATP and the like.

The composition of the present invention may further contain amino acid or amino acid derivative. Examples of the amino acid and amino acid derivative include alanine, valine, leucine, isoleucine, glycine, proline, serine, glutamine, glutamic acid, arginine, lysine, asparagine, aspartic acid, citrulline, ornithine, carnitine and derivatives thereof. These amino acids may be any of L form, D form and a mixture thereof (e.g., racemate).

The composition of the present invention may further contain medium chain fatty acid triglyceride. For easy handling, licorice polyphenol is preferably dissolved in medium chain fatty acid triglyceride before use. The medium chain fatty acid triglyceride in this case is not particularly limited as long as it is constituted by fatty acid having a carbon number of 6 to 12. Triglyceride constituted by saturated fatty acid having a carbon number of 8 or 10 is preferable, and one containing triglyceride constituted by saturated fatty acid having a carbon number of 8 as a main component is more preferable. The constituent ratio of fatty acid of the medium chain fatty acid triglyceride is not particularly limited. The constituent ratio of fatty acid having a carbon atom number of 8 to 10 is preferably not less than 50 wt%, more preferably not less than 70 wt%. Particularly, medium chain fatty acid triglyceride having a specific gravity of 0.94 - 0.96 at 20°C and a viscosity of 23 - 28 cP at 20°C is particularly preferable. Moreover, any of naturally occurring medium chain fatty acid triglyceride, one prepared by transesterification and the like, and the like can be used.
In addition, medium chain fatty acid triglyceride to be used in the present invention may be glycerol fatty acid ester containing medium chain fatty acid triglyceride. Preferred is glycerol fatty acid ester containing not less than 50 wt% of medium chain fatty acid triglyceride, and more preferred is glycerol fatty acid ester containing not less than 70 wt% of medium chain fatty acid triglyceride.

In addition, partial glyceride can also be used concurrently along with the above-mentioned medium chain fatty acid triglyceride. Partial glyceride of medium chain fatty acid can be used without using medium chain fatty acid triglyceride. The partial glyceride is glycerol fatty acid ester containing partial glycerides, preferably glycerol fatty acid ester containing not less than 50 wt% of partial glyceride, more preferably not less than 70 wt% of partial glyceride. The partial glyceride to be used here is diglyceride (1,2-diacylglycerol, 1,3-diacylglycerol) or monoglyceride (1-monoacylglycerol, 2-monoacylglycerol), or a mixture thereof. In view of processability, diglyceride is preferable. As the partial glyceride here, any of naturally occurring partial glyceride, one prepared by transesterification and the like, and the like can be used. The fatty acid residue constituting the above-mentioned partial glyceride is, for example, one having a carbon number of 4 to 24 and, where necessary for the use, saturated fatty acid thereof, unsaturated fatty acid thereof and the like may be employed. For example, when flowability is required, unsaturated fatty acid is preferable, and when plasticity is required, saturated fatty acid is preferable. In addition, branched fatty acid such as isostearic acid and the like can also be used.

A composition containing licorice polyphenol and other active ingredient of the present invention may further contain other materials as appropriate. Such materials are not particularly limited and, for example, excipient, disintegrant, lubricant, binder, antioxidant, colorant, anticoagulant, absorption promoter, solubilizing agents of active ingredient, stabilizer, fat and oil, viscosity modifier and the like can be used.

While the above-mentioned excipient is not particularly limited, for example, sucrose, lactose, glucose, cornstarch, mannitol, crystalline cellulose, calcium phosphate, calcium sulfate, magnesium sulfate and the like can be mentioned.

While the above-mentioned disintegrant is not particularly limited, for example, starch, agar, calcium citrate, calcium carbonate, sodium hydrogen carbonate, dextrin, crystalline cellulose, carboxymethylcellulose, tragacanth and the like can be mentioned.

While the above-mentioned lubricant is not particularly limited, for example, talc, magnesium stearate, polyethylene glycol, silica, hydrogenated vegetable oil and the like can be mentioned.

While the above-mentioned binder is not particularly limited, for example, ethylcellulose, methylcellulose, hydroxypropylmethylcellulose, tragacanth, shellac, gelatin, gum arabic, polyvinylpyrrolidone, polyvinyl alcohol, polyacrylic acid, polymethacrylic acid, sorbitol and the like can be mentioned.

While the above-mentioned antioxidant is not particularly limited, for example, ascorbic acid, tocopherol, sodium bisulfite, thiosodium sulfate, pyrrosodium sulfite, citric acid and the like can be mentioned.

While the above-mentioned colorant is not particularly limited, for example, those permitted for addition to pharmaceutical products and food, and the like can be mentioned.

While the above-mentioned anticoagulant is not particularly limited, for example, stearic acid, talc, light anhydrous silicic acid, hydrated silicon dioxide and the like can be mentioned.

While the above-mentioned absorption promoter is not particularly limited, for example, surfactants such as higher alcohols, higher fatty acids, sucrose fatty acid ester, sorbitan fatty acid ester, sorbitan polyoxyethylene fatty acid ester, polyglycerin fatty acid ester etc., and the like can be mentioned.

While the above-mentioned solubilizing agent for active ingredients is not particularly limited, organic acids such as fumaric acid, succinic acid, malic acid etc., and the like can be mentioned.

While the above-mentioned stabilizer is not particularly limited, for example, benzoic acid, sodium benzoate, ethyl parahydroxybenzoate, propylene glycol and the like can be mentioned.

While the above-mentioned fat and oil component is not particularly limited, for example, vegetable oil such as corn oil, rapeseed oil, high erucic acid rapeseed oil, soybean oil, olive oil, safflower oil, cottonseed oil, sunflower oil, rice bran oil, perilla oil, perilla oil, flaxseed oil, evening primrose oil, cacao butter, peanut oil, palm oil, palm kernel oil and the like, animal oils such as fish oil, beef fat, lard, milk fat, egg yolk oil and the like, fats and oils obtained by fractionation, hydrogenation, transesterification and the like of the above as starting materials, or a mixed oil thereof can be used.

While the above-mentioned viscosity modifier is not particularly limited, for example, beeswax, vegetable wax, lanolin, microcrystalline wax, liquid paraffin and the like can be mentioned.

The composition of the present invention can be used as a body fat accumulation suppressor, a body fat decomposition promoter or an energy production promoter.
The body fat accumulation suppressor of the present invention suppresses ingested lipid, saccharides and the like from being accumulated as body fat.
The body fat decomposition promoter of the present invention promotes decomposition of body fat by activating the fatty acid decomposition system to convert fatty acid into energy and heat in the liver, muscles and the like.
The energy production promoter of the present invention promotes production of energy in the tricarboxylic acid cycle (TCA cycle) and the electron transport system thereafter and the like.
Generally, it is known that anorectic agents, inhibitors of lipid or sugar absorption, body fat synthesis inhibitors, fatty acid metabolism promoters that promote decomposition of body fat, energy production promoters, heat production promoters, low-energy foods and the like are effective for the prophylaxis or treatment of obesity. Anorectic agents and inhibitors of lipid or sugar absorption have an action to suppress accumulation of body fat by decreasing the calories ingested into the body. Body fat synthesis inhibitors suppress accumulation of ingested fatty acid as body fat by suppression of re-synthesis of fatty acid by suppressing the fatty acid sysnthesis system. Fatty acid metabolism promoters activate the fatty acid decomposition system, and convert fatty acid into energy and heat in the liver and muscles, whereby a body fat decomposition promoting action is achieved. As regards the promotion of energy production, ATP generated in the tricarboxylic acid cycle (TCA cycle) and subsequent electron transport system becomes energy. Thus, a component that activates this pathway has an energy production promoting action. For example, α-lipoic acid, L-carnitine and the like are considered having an action thereof. For promotion of heat production, for example, the action of agonists to β adrenoceptor and the effect of uncoupling protein (UCP) activator are effective. The β3 adrenoceptor agonists and the uncoupling protein activators are known to have a heat production promoting effect and a lipolysis promoting effect. Accordingly, suppression of body fat accumulation and promotion of body fat decomposition are effective for the prophylaxis and/or improvement of lifestyle-related diseases such as obesity and the like.

The body fat accumulation suppressor of the present invention is desirably ingested in an amount of 0.01 - 100 mg/kg body weight, preferably 0.1 - 30 mg/kg body weight, as a total amount of licorice polyphenol, by an adult per day.
The body fat decomposition promoter of the present invention is desirably ingested in an amount of 0.01 - 100 mg/kg body weight, preferably 0.1 - 30 mg/kg body weight, as a total amount of licorice polyphenol, by an adult per day.
The energy production promoter of the present invention is desirably ingested in an amount of 0.01 - 100 mg/kg body weight, preferably 0.1 - 30 mg/kg body weight, as a total amount of licorice polyphenol, by an adult per day.

While a daily dose of other active ingredients to be ingested by an adult varies depending on each component, an antioxidant ingredient is desirably ingested in an amount of 0.001 - 1000 mg/kg body weight, preferably 0.01 - 100 mg/kg body weight.
Similarly, a daily dose of polyphenol other than licorice polyphenol to be ingested is desirably 0.001 - 1000 mg/kg body weight, preferably 0.01 - 100 mg/kg body weight.
Furthermore, a dose of the component relating to absorption or metabolism of lipid is desirably 0.001 - 1000 mg/kg body weight, preferably 0.01 - 100 mg/kg body weight.

The composition of the present invention can be used for the prophylaxis, improvement or treatment of obesity and metabolic syndrome, diabetes, hyperlipidemia or hypertension caused by the obesity. When the composition of the present invention is used for the prophylaxis, improvement or treatment of visceral fat accumulation, obesity, metabolic syndrome, diabetes, hyperlipidemia or hypertension, it is desirably ingested in such an amount as to enable ingestion of a total amount of licorice polyphenol of 0.01 - 100 mg/kg body weight, preferably 0.1 - 30 mg/kg body weight, by an adult per day, though subject to change depending on the administration subject, administration route, target disease, symptom and the like.
In addition, while the amount of other active ingredients ingested by an adult per day varies depending on the administration subject, administration route, target disease, symptom and the like, an antioxidant ingredient is desirably ingested in an amount of 0.001 - 1000 mg/kg body weight, preferably 0.01 - 100 mg/kg body weight.
Similarly, the amount of polyphenol other than licorice polyphenol to be ingested is desirably 0.001 - 1000 mg/kg body weight, preferably 0.01 - 100 mg/kg body weight.
Furthermore, the ingestion amount of a component relating to absorption or metabolism of lipid is desirably 0.001 - 1000 mg/kg body weight, preferably 0.01 - 100 mg/kg body weight.
The above-mentioned ingestion dose can be taken at once or in several portions a day. While the ingestion time is not particularly limited, it is generally not less than 2 weeks, preferably not less than 1 month.

The composition of the present invention is useful for application to human, animals other than human [e.g., mammals except human (livestock and pet animals such as swine, bovine, horse, dog etc.), birds (poultry and pet animals such as turkey, chicken etc.), etc.] and the like. Preferably, the composition is applied to obese human and obese animals other than human, or patients with metabolic syndrome, diabetes, hyperlipidemia or hypertension caused by obesity, subjects pointed out to have a risk of developing such diseases, and healthy subjects desirous of the prophylaxis thereof.

The composition of the present invention can be used for the treatment of obesity, metabolic syndrome, diabetes, hyperlipidemia or hypertension.

The composition of the present invention can also be used in combination with pharmaceutical products (sometimes to be referred to as concomitant drug) used for the treatment of obesity, metabolic syndrome, diabetes, hyperlipidemia or hypertension. The "combination" here means not only formation of a single preparation but also combined ingestion of separate preparations simultaneously or in a staggered manner.
The dose of the concomitant drug can be appropriately determined based on the dose clinically employed for each pharmaceutical agent. In addition, the mixing ratio of the composition of the present invention and the concomitant drug can be appropriately determined according to the administration subject, administration route, target disease, symptom, combination and the like.

Examples of the pharmaceutical product used for the treatment of obesity and metabolic syndrome caused by the obesity include, but are not limited to, fenfluramine, fluoxetine, mazindol and the like as pharmaceutical products that suppress appetite, and acarbose, voglibose, lipase inhibitor and the like as pharmaceutical products that inhibit digestion and absorption.
Examples of the pharmaceutical product used for the treatment of diabetes include, but are not limited to, insulin preparations, sulfonylureas, α-glucosidase inhibitors, biguanides, insulin sensitizers, insulin action enhancers, rapid-acting postprandial hypoglycemic drugs and the like, which are used as general diabetes drugs.
Examples of the pharmaceutical products used for the treatment of hyperlipidemia include, but are not limited to, statin drugs, anion exchange resins, probucols, fibrates, icosapentate, ethyl nicotinate and the like.
Examples of the pharmaceutical products used for the treatment of hypertension include, but are not limited to, diuretics, β-blockers, α1-blockers, calcium antagonists, angiotensin converting enzyme inhibitors, angiotensin II antagonists and the like.

The composition of the present invention can be used as foods such as functional food, nutritional supplement and the like, drinks, pharmaceutical products, quasi-drugs, cosmetics, animal drugs, pet foods, feeds or prey feeds.

When the composition of the present invention is used as a functional food, nutritional supplement, pharmaceutical product, quasi-drug or animal drug, it may be directly ingested. In addition, the composition of the present invention may be in the form of an oral preparation. Examples of the oral preparation include a dosage form that can be taken orally, such as capsules (hard capsule, microcapsule, soft capsule), tablets, powders, chewable preparations, syrup, liquid and the like. A substrate for capsules is not particularly limited, and gelatin derived from beef bone, bovine hide, pig hide, fishskin and the like, other substrates, for example, seaweed-derived products such as carageenan, alginic acid and the like, vegetable seed-derived products such as locust bean gum, guar gum and the like, microorganism-derived products such as pullulan, curdlan and the like and agents used for production such as celluloses, which are usable as food additives, can also be used.

In addition, the composition of the present invention can be added to general foods. For example, the composition can be used by appropriately adding to drinks such as milk beverage, beverage, energy drink, beauty drink and the like, confectionery such as chewing gum, chocolate, candy, jelly, cake, biscuit, cracker and the like, frozen desserts such as ice cream, ice desserts and the like, noodles such as Japanese wheat noodles, Chinese noodles, spaghetti, instant noodles and the like, processed seafood paste products such as fish cake, tube-like fish sausage, square-shaped fish cake and the like, seasoning such as dressing, mayonnaise, sauce and the like, various retort foods such as bread, ham, rice soup, rice, soup, various frozen foods and the like. Needless to say, the composition can be utilized as other food forms, and can be further utilized for pet foods, livestock feeds and the like.

The composition of the present invention can be used for foods, drinks, functional foods, nutritional supplements, pharmaceutical products, quasi-drugs, cosmetics, animal drugs, pet foods, feeds or prey feeds. The functional foods here refers to any food form other than pharmaceutical products, which can be ingested for maintaining health, such as what is called health foods, health supplements, specified health foods, foods with nutrient function claims and the like. In addition, when the food is what is called a health food, a form containing licorice polyphenol and other active ingredients in unit ingestion amounts per meal and the like can be employed. When the food is a health drink, examples thereof include a drink obtained by suspending or dissolving licorice polyphenol and other active ingredients, which is packed in a bottle etc. for a single consumption.
The unit ingestion amount per meal in the case of food is the amount of active ingredient to be ingested. Particularly, in the case of food, the amount of the whole ingested food is different for each individual, and the content of the active ingredient in the composition cannot be easily defined in general. Therefore, definition of an amount of an active ingredient for one time ingestion, in consideration of the aforementioned effective ingestion amount per day, is recommended. The one time ingestion amount varies as appropriate depending on the age, body weight, sex, stress level and the like. In the case of pharmaceutical products, they contain an active ingredient in a one-time ingestion amount, i.e., an amount to be administered by one dose, in a package or bottle for a single consumption and the like.

Furthermore, the composition of the present invention may be in the form of a parenteral agent. For example, a form for direct application to the skin may be employed. In this case, the dosage form is not particularly limited and examples thereof include cream, paste, jelly, gel, emulsion and liquid obtained by dissolving or dispersing the above-mentioned composition in a suitable base (ointment, liniment, lotion, spray etc.), those obtained by dissolving or dispersing the above-mentioned drug in a base and spreading the same on a support (patch etc.), and those obtained by dissolving or dispersing the above-mentioned composition in an adhesive and spreading the same on a support (plaster, tape etc.).

When the composition of the present invention is used as a quasi-drug, the quasi-drug refers to those defined in the Pharmaceutical Affairs Law, and includes oral preparations (liquids such as extract, elixir, syrup, tincture, lemonade etc., solid agents such as capsule, granule, pill, powder, tablet etc.) and the like.

A method of promoting energy production, a method for the prophylaxis, improvement or treatment of obesity, metabolic syndrome, diabetes, hyperlipidemia or hypertension, and a method of becoming slim, which comprise administering an effective amount of the composition of the present invention to a subject, are also among the embodiments of the present invention. The method of becoming slim according to the present invention comprises reducing the body weight by mainly decreasing the body fat.

Use of licorice polyphenol and other components for the production of the composition of the present invention used for suppressing body fat accumulation, promoting body fat decomposition or promoting energy production is also a still another embodiment of the present invention.

### Examples

The present invention is explained in more detail in the following by referring to Examples, which are not to be construed as limitative.

### (Example 1)

Rhizome (1.0 Kg) of licorice (G. glabra) from Afghan was extracted (45°C, 2 hr, twice) with ethanol (5.0 L) and concentrated under reduced pressure to give a concentrated liquid (0.45 L). Then, the concentrated liquid (0.3 L) was treated with activated carbon and further concentrated to give a licorice hydrophobic extract-containing ethanol solution (123.6 g, containing 24.8 g of licorice hydrophobic extract). The licorice hydrophobic extract-containing ethanol solution (50.0 g) was concentrated under reduced pressure to give a licorice hydrophobic extract (10.0 g).

### (Example 2)

The licorice hydrophobic extract-containing ethanol solution (63.9 g) of Example 1 and medium chain fatty acid triglyceride (Actor M2; Riken Vitamin Co., Ltd., fatty acid composition C8:C10=99:1, 18.8 g) were mixed, and the mixture was concentrated under reduced pressure to remove ethanol. 28.7 g obtained by concentration under reduced pressure was suction filtered to remove an insoluble material, which was washed with hexane. The resulting recovered oil was added to the earlier filtrate. Medium chain fatty acid triglyceride (4.5 g) was added to the recovered filtrate (26.2 g) to give a licorice hydrophobic extract-containing medium chain fatty acid triglyceride solution (30.7 g, containing 8.9 g of licorice hydrophobic extract).

### HPLC analysis

### <Preparation of HPLC analysis sample>

The above-mentioned licorice hydrophobic extract-containing medium chain fatty acid triglyceride solution (1 g) was dissolved in methanol for HPLC, and the total amount was adjusted to 100 ml.

### <HPLC conditions>

column: YMC, J'sphere ODS-H80, 4.6×250 mm column temperature: 40°C
mobile phase: A=20 mM aqueous phosphoric acid solution
   B=acetonitrile:methanol (50:50=v/v)
gradient: conditions under which the ratio of B relative to mobile phase A was maintained constant at 50% for 20 minutes from the start of the analysis, raised at a given rate to reach 80% in 75 minutes after the 20 minutes, maintained constant at 100% from 75 minutes to 80 minutes and maintained constant at 50% from 80 minutes to 100 minutes
flow rate: 1 ml/min
wavelength: UV 282 nm
sample injection volume: 20 µL

### <Analysis results>

The content of each component contained in 1 g of the licorice hydrophobic extract-containing medium chain fatty acid triglyceride solution was glabrene (4.4 mg), glabridin (30.0 mg), glabrol (6.0 mg), and 4'-O-methylglabridin (5.2 mg).

### <Polyphenol analysis>

As a result of the polyphenol content measurement by Folin-Denis assay and using glabridin (manufactured by Wako Pure Chemical Industries, Ltd.) as a standard substance, the total content of polyphenol in 1 g of the licorice hydrophobic extract-containing medium chain fatty acid triglyceride solution was 239.1 mg.

### (Reference Example)

Rhizome (40 g) of licorice (G. glabra) from Afghan was extracted (45°C, 2 hr, twice) with 80% ethanol (300 mL), concentrated under reduced pressure, treated with activated carbon, and further concentrated under reduced pressure to remove ethanol, whereby a licorice hydrophobic extract (5.2 g) was obtained. The contents of glabridin and glycyrrhizin were glabridin (2.0 wt%) and glycyrrhizin (7.1 wt%) relative to the licorice hydrophobic extract.
The glycyrrhizin content of the licorice hydrophobic extract-containing medium chain fatty acid triglyceride solution of Example 2 was measured under the same conditions to find that the content was not more than the detection limit (0.001 wt%).

### <HPLC conditions for glycyrrhizin>

column: YMC, J'sphere ODS-H80, 4.6×250 mm
column temperature: 40°C
mobile phase: A=acetonitrile
   B=20 mM aqueous phosphoric acid solution
gradient: conditions under which the ratio of mobile phase A was maintained constant at 36% for 10 minutes from the start of the analysis, raised at a given rate to reach 45% in 50 minutes after the 10 minutes, maintained constant at 100% from 50 minutes to 55 minutes and maintained constant at 36% from 55 minutes to 75 minutes
flow rate: 1 ml/min
wavelength: UV 254 nm
sample injection volume: 20 µL

### (Example 3)

Rhizome (500 g) of licorice (G. glabra) from Afghan was extracted (45°C, 2 hr, twice) with 91% ethanol (3.1 L), concentrated under reduced pressure, treated with activated carbon, and further concentrated under reduced pressure to remove ethanol, whereby a licorice hydrophobic extract (42.5 g) was obtained. The contents of glabridin and glycyrrhizin were glabridin (3.6 wt%) and glycyrrhizin (2.1 wt%) relative to the licorice hydrophobic extract.

### (Example 4)

To a mixture of beeswax (8 parts by weight), lecithin (2 parts by weight) and diglycerol monooleate (18 parts by weight) were added the licorice hydrophobic extract-containing medium chain fatty acid triglyceride solution (70 parts by weight) obtained in Example 2, astaxanthin (1 part by weight) and vitamin A (1 part by weight) and the mixture was mixed and pressed into a gelatin film by a rotary soft capsule production apparatus to give an eatable and drinkable soft capsule.

### (Example 5)

The licorice hydrophobic extract (10 parts by weight) obtained in Example 1, α-lipoic acid (5 parts by weight), vitamin C (1 part by weight), vitamin E (2 parts by weight), L-valine (2 parts by weight), aspartic acid (2 parts by weight), L-carnitine (5 parts by weight), catechin (2 parts by weight), sesamin (2 parts by weight), citric acid (2 parts by weight), fructooligosaccharide (2 parts by weight), beeswax (5 parts by weight), soybean peptide (5 parts by weight) and medium chain fatty acid triglyceride (55 parts by weight) were mixed and pressed into a gelatin film by a rotary soft capsule production apparatus to give an eatable and drinkable soft capsule.

### (Example 6)

The licorice hydrophobic extract-containing medium chain fatty acid triglyceride solution (5 parts by weight) obtained in Example 2 and lycopene (tomato extract, 1 parts by weight) were adsorbed onto microcrystalline cellulose (40 parts by weight), and mixed with cornstarch (20 parts by weight), lactose (8 parts by weight), carboxymethylcellulose (8 parts by weight) and magnesium stearate (2 parts by weight). Then, an aqueous polyvinylpyrrolidone solution (8 parts by weight) was added to granulate the mixture. Talc (8 parts by weight) was added as a lubricant and the mixture was tableted.

### (Example 7)

A cream containing licorice hydrophobic extract (10 parts by weight) obtained in Example 1, astaxanthin (1 part by weight), glycerolsorbitan fatty acid ester (60 parts by weight), microcrystalline wax (10 parts by weight), olive oil (30 parts by weight), liquid paraffin (180 parts by weight), magnesium stearate (10 parts by weight), propylene glycol (37 parts by weight), magnesium sulfate (7 parts by weight) and dechlorinated water (655 parts by weight) was prepared.

### (Example 8) Preparation of in vitro obese model

As adipocyte precursor cells, 3T3-L1 cells (Dainippon Sumitomo Pharmaceuticals Co., Ltd.) were used. The 3T3-L1 cells were cultured in passage medium A (10% bovine serum-added DMEM supplemented with penicillin streptomycin) in a 75 cm² flask. The 3T3-L1 cells were plated in a 24 well plate at 20000 cells/well. The next day, the medium was changed to passage medium B (10% fetal calf serum-added high glucose DMEM supplemented with penicillin·streptomycin) and, 48 hr later, the medium was changed to differentiation induction medium. The differentiation induction medium contained passage medium B as a base medium, and dexamethasone, isobutylmethylxanthine, insulin and pioglitazone at a final concentration of 0.5 µM, 500 µM, 8 µg/ml and 2 µM, respectively. Further, 48 hr later, the medium was changed to a differentiation promoting medium. The differentiation promoting medium contained passage medium B as a base medium, and insulin and pioglitazone at a final concentration of 8 µg/ml and 2 µM, respectively. 48 hr later, the medium was changed to passage medium B. Thereafter, the medium was changed to passage medium B every other day. 7 to 10 days later, differentiation of 3T3-L1 cells into mature adipocyte cells were confirmed with a phase contrast microscope, and the cells were subjected to the following test.

### (Example 9) Lipid decomposition promoting action of licorice polyphenol and antioxidant ingredient

Using the in vitro obesity model prepared in Example 9, the effects of a licorice hydrophobic extract containing licorice polyphenol as a main component and antioxidant ingredient on lipid decomposition were studied. In addition, the effects of each combined use were also studied.

After removing passage medium B by an aspirator, the cells were washed with KRB-HEPES (128 mM NaCl, 10 mM NaH₂PO₄, 4.7 mM KCl, 1.25 mM CaCl₂, 1.25 mM MgCl₂, 50 mM HEPES, pH 7.4). The licorice hydrophobic extract (50 mg/ml) of Example 1 and α-lipoic acid (100 mM) as an antioxidant ingredient were each dissolved in DMSO. The licorice hydrophobic extract and α-lipoic acid were diluted to a desired concentration with a solution (Buffer A) obtained by adding BSA (final concentration 1%), glucose (final concentration 5 mM) and epinephrine (final concentration 0.1 µM) to KRB-HEPES, and added to the cells. The final concentration of the licorice hydrophobic extract was adjusted to 25 µg/ml, and the final concentration of α-lipoic acid was adjusted to 30 µM, and each of them or both of them were added. The culture supernatant was recovered 24 hr later, and the centrifuged supernatant was used as a sample. The glycerol concentration was measured using Glycerol Reagent A manufactured by Zen-Bio, and the glycerol amount per protein concentration of the cell was calculated and compared. A glycerol secretion amount is generally used as an index of lipid decomposition in the cell, where a higher amount thereof means stronger lipid decomposition promoting effect.

As is clear from Table 1, the combined use of a licorice hydrophobic extract and α-lipoic acid afforded a remarkable lipid decomposition promoting effect.

**Table 1**

| sample | glycerol secretion amount (µM/mg protein) |
|---|---|
| control | 92.6±12.3 |
| licorice hydrophobic extract (25 µg/ml) | 107.6±5.4 |
| α-lipoic acid (30 µM) | 89.6±4.8 |
| licorice hydrophobic extract (25 µg/ml)+ α-lipoic acid (30 µM) | 112.4±4.1* |

| | |
|---|---|
| * T-test vs control, P<0.05 | |

### (Example 10) Lipid decomposition promoting action of licorice polyphenol and polyphenol other than licorice polyphenol

Using the in vitro obesity model prepared in Example 9, the effects of a licorice hydrophobic extract containing licorice polyphenol as a main component and polyphenol other than licorice polyphenol on lipid decomposition were studied. In addition, the effects of each combined use were also studied.

After removing passage medium B, the cells were washed with KRB-HEPES. The licorice hydrophobic extract (50 mg/ml) of Example 1 and genistein (100 mM) as polyphenol other than licorice polyphenol were each dissolved in DMSO. The licorice hydrophobic extract and genistein were diluted to a desired concentration with Buffer A, and added to the cells. The final concentration of the licorice hydrophobic extract was adjusted to 25 µg/ml, and the final concentration of genistein was adjusted to 10 µM, and each of them or both of them were added. The culture supernatant was recovered 24 hr later, and the centrifuged supernatant was used as a sample. The glycerol concentration was measured using Glycerol Reagent A manufactured by Zen-Bio, and the glycerol amount per protein concentration of the cell was calculated and compared.

As is clear from Table 2, the combined use of a licorice hydrophobic extract and genistein afforded a remarkable lipid decomposition promoting effect.

**Table 2**

| sample | glycerol secretion amount (µM/mg protein) |
|---|---|
| control | 100.3±13.1 |
| licorice hydrophobic extract (25 µg/ml) | 110.9±12.3 |
| genistein (10 µM) | 111.1±6.3 |
| licorice hydrophobic extract (25 µg/ml)+ genistein (10 µM) | 126.0±5.7* |

| | |
|---|---|
| * T-test vs control, P<0.05 | |

### (Example 11) Lipid decomposition promoting action of licorice polyphenol and component modifying lipid metabolism in cell

Using the in vitro obesity model prepared in Example 9, the effects of a licorice hydrophobic extract containing licorice polyphenol as a main component and a component modifying lipid metabolism in cell on lipid decomposition were studied. In addition, the effects of each combined use were also studied.

After removing passage medium B, the cells were washed with KRB-HEPES. The licorice hydrophobic extract (50 mg/ml) of Example 1 and carnitine (100 mM) as a component modifying lipid metabolism were each dissolved in DMSO. The licorice hydrophobic extract and carnitine were diluted to a desired concentration with Buffer A, and added to the cells. The final concentration of the licorice hydrophobic extract was adjusted to 25 µg/ml, and the final concentration of carnitine was adjusted to 80 µM, and each of them or both of them were added. The culture supernatant was recovered 24 hr later, and the centrifuged supernatant was used as a sample. The glycerol concentration was measured using Glycerol Reagent A manufactured by Zen-Bio, and the glycerol amount per protein concentration of the cell was calculated and compared.

As is clear from Table 3, a combined use of a licorice hydrophobic extract with carnitine afforded a remarkable lipid decomposition promoting effect.

**Table 3**

| sample | glycerol secretion amount (µM/mg protein) |
|---|---|
| control | 52.1±4.1 |
| licorice hydrophobic extract (25 µg/ml) | 50.8±1.4 |
| carnitine (80 µM) | 43.5±1.9 |
| licorice hydrophobic extract (25 µg/ml)+ carnitine (80 µM) | 60.8±4.7* |

| | |
|---|---|
| * T-test vs control, P<0.05 | |

While some of the embodiments of the present invention have been described in detail in the above, those of ordinary skill in the art can make various modifications and changes to the particular embodiments shown without substantially departing from the novel teaching and advantages of the present invention. Such modifications and changes are encompassed in the spirit and scope of the present invention as set forth in the appended claims.

This application is based on a patent application No. 2007-131779 filed in Japan and US provisional application No. 60/950432, the contents of which are incorporated in full herein by this reference.

## Claims

1. A composition comprising licorice polyphenol and an antioxidant ingredient.

2. The composition of claim 1, wherein the antioxidant ingredient is at least one kind selected from the group consisting of α-lipoic acid, glutathione, astaxanthin, lycopene, taurine, methionine, S-adenosylmethionine, cysteine, cystine, thiol compound, reducing sugar, sesamin, capsaicin, citric acid, pycnogenol, fucoxanthin, lignan, piperine, policosanol, anthocyanin, proanthocyanidin, chlorogenic acid, vitamin C, vitamin A, vitamin E and carotenoid.

3. A composition comprising licorice polyphenol and polyphenol other than licorice polyphenol.

4. The composition of claim 3, wherein the polyphenol other than licorice polyphenol is at least one kind selected from the group consisting of genistein, daidzein, quercetin, rutin, catechin, epigallocatechin gallate, hesperidin, nobiletin, tyrosol, hydroxytyrosol, oleuropein, naringenin, caffeic acid, apple polyphenol, tea polyphenol and gallic acid.

5. A composition comprising licorice polyphenol and a component relating to absorption or metabolism of lipid.

6. The composition of claim 5, wherein the component relating to the absorption or metabolism of lipid is a lipid absorption inhibitory component.

7. The composition of claim 6, wherein the lipid absorption inhibitory component is at least one kind selected from the group consisting of lactobacillus, yeast, polysaccharides, dietary fiber, oligosaccharide, inulin, chitin, chitosan, phytosterol, pectin, glucosamine, N-acetylglucosamine, hyaluronan, chondroitin sulfate, sodium alginate and tannin.

8. The composition of claim 5, wherein the component relating to the absorption or metabolism of lipid is a component modifying lipid metabolism in cells.

9. The composition of claim 8, wherein the component modifying lipid metabolism in cells is at least one kind selected from the group consisting of carnitine, capsaicin, malic acid, hydroxycitric acid, acetic acid, propionic acid, citric acid, sesamin, pyruvic acid, curcuminoid, gingerol, pycnogenol, resveratrol, soybean protein, conjugated linoleic acid, catechin, caffeine, fucoxanthin, diacylglycerol, glucomannan, lignan, fructooligosaccharide, piperine, policosanol, chrome, zinc, vanadium, phospholipid and highly-unsaturated fatty acid.

10. The composition of claim 8, wherein the component modifying lipid metabolism in cells is at least one kind selected from the group consisting of chili pepper, tea, fermented tea, coffee, pepper, garcinia cambogia, ginger, blueberry, citrus, grape and extracts thereof.

11. The composition of any one of claims 1 - 10, further comprising vitamin or a vitamin-like component.

12. The composition of claim 11, wherein the vitamin or vitamin-like component is at least one kind selected from the group consisting of vitamin B, vitamin K, folic acid and inositol.

13. The composition of any one of claims 1 - 12, further comprising an amino acid or an amino acid derivative.

14. The composition of claim 13, wherein the amino acid or amino acid derivative is at least one kind selected from the group consisting of alanine, valine, leucine, isoleucine, glycine, proline, glutamine, glutamic acid, asparagine, aspartic acid, arginine, lysine, citrulline, ornithine and derivatives thereof.

15. The composition of any one of claims 1 - 14, comprising licorice polyphenol in the form of a licorice hydrophobic extract.

16. The composition of any one of claims 1 - 15, wherein the licorice polyphenol comprises at least glabridin.

17. The composition of claim 16, wherein the content of glycyrrhizin in the composition is not more than the content of glabridin.

18. The composition of any one of claims 1 - 17, further comprising medium chain fatty acid triglyceride.

19. The composition of any one of claims 1 - 18, which is a body fat accumulation suppressor.

20. The composition of any one of claims 1 - 18, which is a body fat decomposition promoter.

21. The composition of any one of claims 1 - 18, which is an energy production promoter.

22. The composition of any one of claims 1 - 21, which is used for the treatment of obesity, metabolic syndrome, diabetes, hyperlipidemia or hypertension.

23. The composition of any one of claims 1 - 22, which is used for a food, drink, functional food, nutritional supplement, pharmaceutical product, quasi-drug, cosmetic, animal drug, pet food, feed or prey feed.

24. The composition of any one of claims 1 - 23, which is used in combination with a pharmaceutical product to be used for the treatment of obesity, metabolic syndrome, diabetes, hyperlipidemia or hypertension.

25. The composition of any one of claims 1 - 24, which is a preparation for oral administration.

26. A method for promoting an energy production, comprising administering an effective amount of the composition of any one of claims 1 - 25 to a subject.

27. A method for the prophylaxis, improvement or treatment of obesity, metabolic syndrome, diabetes, hyperlipidemia or hypertension, comprising administering an effective amount of the composition of any one of claims 1 - 25 to a subject.

28. A method for becoming slim, comprising administering an effective amount of the composition of any one of claims 1 - 25 to a subject.

29. Use of licorice polyphenol and an antioxidant ingredient for the production of the composition of any one of claims 1 - 25 for suppressing body fat accumulation, promoting body fat decomposition or promoting energy production.

30. Use of licorice polyphenol and polyphenol other than licorice polyphenol for the production of the composition of any one of claims 1 - 25 for suppressing body fat accumulation, promoting body fat decomposition or promoting energy production.

31. Use of licorice polyphenol and a component relating to absorption or metabolism of lipid for the production of the composition of any one of claims 1 - 25 for suppressing body fat accumulation, promoting body fat decomposition or promoting energy production.
